## Europäisches Patentamt
## European Patent Office
## Office européen des brevets

(19)

(11) Veröffentlichungsnummer: **0 382 128 B1**

(12)

# EUROPÄISCHE PATENTSCHRIFT

(45) Veröffentlichungstag der Patentschrift: **09.08.95**

(51) Int. Cl.6: **A61L 15/24**, A61L 15/32, A61L 15/28

(21) Anmeldenummer: **90102173.3**

(22) Anmeldetag: **03.02.90**

(54) **Vernetzte Hydrogele und ihre Verwendung als Wundauflage.**

(30) Priorität: **08.02.89 DE 3903672**

(43) Veröffentlichungstag der Anmeldung:
**16.08.90 Patentblatt 90/33**

(45) Bekanntmachung des Hinweises auf die
Patenterteilung:
**09.08.95 Patentblatt 95/32**

(84) Benannte Vertragsstaaten:
**AT BE CH DE DK ES FR GB GR IT LI LU NL SE**

(56) Entgegenhaltungen:
**EP-A- 0 174 849**
**DE-A- 2 849 570**
**GB-A- 2 080 814**
**GB-A- 2 131 701**
**US-A- 4 243 656**

(73) Patentinhaber: **Lohmann GmbH & Co. KG**
**Irlicher Strasse 55**
**D-56567 Neuwied (DE)**

(72) Erfinder: **Czech, Zbigniew, Dr., Dipl. Chem.**
**Rostocker Strasse 10**
**D-5400 Koblenz 1 (DE)**
Erfinder: **Seeger, Kurt, Dr., Dipl.-Chem.**
**Matthias-Claudius-Strasse 3**
**D-5450 Neuwied 12 (DE)**

(74) Vertreter: **Flaccus, Rolf-Dieter, Dr.**
**Patentanwalt**
**Sperlingsweg 32**
**D-50389 Wesseling (DE)**

**Beschreibung**

Die Erfindung betrifft vernetzte Hydrogele auf Basis mehrwertiger Alkohole, Biopolymere und synthetischer Polymere als medizinische Wundauflagen.

In den letzten Jahren kamen eine Reihe von Hydrogel/-kolloid-Systemen als Wundauflagen auf den Markt, die ein hohes Absorptionsvermögen für Wundsekrete aufweisen, so daß ein mehrtägiges Verbleiben auf der Wunde mit dem Vorteil einer ungestörten Wundheilung unter Ausschluß von Bakterien möglich ist.

Beispiele für solche hydrogelartige Materialien sind anhand folgender Patenschriften beschrieben:

| | |
|---|---|
| CA-A 1 180 622 | Gelatine + Polyethylenoxid + Polyethylenimin |
| DE-C 28 49 570 | Hydrophiles Poly(meth-)acrylsäurederivat in Gegenwart von Polysaccharid/Protein |
| DE-C 30 31 304 | Basis: hydrophile ethylenisch ungesättigte Monomere, vernetzt mit difunktionellen Verbindungen |
| EP-B 0 099 758 | synthetische Kollagene oder Alginate und andere Biopolymere |
| EP-B 0 262 405 | Polynatriumacrylat/Polyacrylsäure /Acryloylamid und andere Acrylamidderivate |
| EP-B 0 272 074 | Copolymere aus ungesättigten, carboxylgruppenhaltigen Monomeren + Di- oder Oligosaccharide |
| US-A 3,249,109 | Gelatine, Wasser, mehrwertige Alkohole, Pektin |
| US-A 4,243,656 | Polyacrylatdispersion + Feuchtigkeitsabsorber, Gelatine, Wasser |

Deartig hergestellte Wundauflagen weisen jedoch bei der praktischen Anwendung eine Reihe von Nachteilen auf.

Hydrogele auf Biopolymerbasis haben nach Wundsekretabsorption eine nicht ausreichende mechanische Festigkeit, da die dort vorhandene rein physikalische Vernetzung bei hoher Absorption keine genügende Stabilität liefert. Sie lösen sich bei Körpertemperatur im Wundsekret auf und lassen sich dadurch nicht rückstandsfrei von der Wunde entfernen. Außerdem sind solche Hydrogele nicht transparent, so daß eine Beobachtung der Wunde ohne Verbandwechsel nicht möglich ist.

Hydrogele aus synthetischen Polymeren neigen dagegen zum nicht kontinuierlichen Verlauf der Wundsekretaufnahme. Die Absorptionskapazität ist dabei schon nach relativ kurzer Zeit (ca. 2-3 h) erschöpft.

Diese Ausführungen betreffen auch Produkte nach DE-C 28 49 570 und US-A 3,249,109, die als nächstliegender Stand der Technik zu betrachten sind.

Aufgabe der Erfindung ist es daher, wundsekretabsorbierende Hydrogele herzustellen, die die oben erwähnten Nachteile nicht mehr zeigen.

Die Aufgabe wird erfindungsgemäß durch vernetzte Hydrogele auf Basis einer Kombination aus mehrwertigen Alkoholen, Biopolymeren und synthetischen Polymeren gelöst, wobei Biopolymere und synthetisches Polymer miteinander vernetzt sind.

Gegenstand der Erfindung ist somit eine wundsekretabsorbierende, transparente, folienartige, elastische Wundauflage, erhältlich aus mehrwertigen Alkoholen, Biopolymeren und synthetischen Polymeren und Vernetzung der Biopolymeren mit den synthetischen Polymeren.

Die erfindungsgemäßen Hydrogele sind aus folgenden Bestandteilen aufgebaut:

a) 20 bis 70 Gew.-% wenigstens eines mehrwertigen Alkohols, ausgewählt aus der Gruppe bestehend aus Glycerin, Ethylenglykol, Diethylenglykol, 1,2-Propandiol, 1,3-Propandiol, 1,2-Butandiol, 1,3-Butandiol, 2,3-Butandiol, 1,4-Butandiol, Glycerinmonoacetat oder ein Gemisch dieser Alkohole,

b) 10 bis 35 Gew.-% wenigstens eines natürlichen Gelierungsmittels (Biopolymer), ausgewählt aus der Gruppe, bestehend aus Collagen, Gelatine, Pektine oder deren Gemische oder ein Gemisch Gelatine/Natriumalginat im Verhältnis von 30:1 bis 5:1,

c) 0,05 bis 12 Gew.-% wenigstens eines unvernetzten Copolymeren aus einer oder mehreren Vinylcarbonsäuren und wenigstens einem ihrer Alkali- oder Ammoniumsalze (synthetisches Polymer),

d) 0,05 bis 10 Gew.-% eines dreidimensionale Netzwerke aus bio- und synthetischen Polymeren erzeugenden Vernetzungsmittels, und

e) 0 bis 50 Gew.-% Wasser oder physiologische Kochsalzlösung.

Als mehrwertiger Alkohol wird Glycerin bevorzugt, der allein oder im Gemisch mit weiteren mehrwertigen Alkoholen eingesetzt werden kann.

Andere mehrwertige Alkohole sind Ethylenglykol, Diethylenglykol, 1,2-Propandiol, 1,3-Propandiol, 1,2-Butandiol, 1,3-Butandiol, 2,3-Butandiol, 1,4-Butandiol, Glycerinmonoacetat oder ein Gemisch dieser Alkohole.

Als natürliches Gelierungsmittel (Biopolymer) wird in erster Linie Gelatine allein oder im Gemisch mit anderen Biopolymeren, bevorzugt Alginaten, eingesetzt. Besonders bevorzugt wird eine Kombination aus Gelatine und Natriumalginat im Gewichtsverhältnis von 5:1 bis 30:1.

Als weitere Biopolymere, die allein oder im Gemisch mit Gelatine eingesetzt werden, sind Kollagene und Pektine zu nennen.

Das als synthetisches Polymer eingesetzte unvernetzte Copolymer ist aus wenigstens einer Vinylcarbonsäure und wenigstens einem ihrer Alkali- oder Ammoniumsalze aufgebaut. Als Vinylcarbonsäuren werden Acrylsäure, Methacrylsäure und/oder β-Acryloyloxypropionsäure bevorzugt. Andere geeignete Vinylcarbonsäuren sind Vinylessigsäure, Maleinsäure, Fumarsäure, Crotonsäure, Aconitsäure, Itaconsäure und Gemische dieser Säuren.

Die erfindungsgemäß verwendeten Vernetzungsmittel sind bevorzugt aus der Gruppe der Metallchelate, Orthotitansäureester, Epoxide, Aziridine, Triazine oder Melamin-Formaldehyd-Harze ausgewählt. Besonders bevorzugt sind hier Aziridine und die Gruppe der Metallchelate, z.B. die Acetylacetonate, z.B. die Übergangsmetallacetylacetonate wie Titan- oder Zirkonacetylacetonat. Das Vernetzungsmittel bewirkt die Vernetzung von Biopolymer mit synthetischem Polymer zu bevorzugt dreidimensionalen Netzwerken.

Das erfindungsgemäße wundsekretabsorbierende Hydrogel wird hergestellt, indem die Komponenten, gegebenenfalls unter Rühren und Erwärmen, homogenisiert werden. Dabei ist es zweckmäßig, den Vernetzer der bereits homogen vermischten Masse der übrigen Komponenten zuzusetzen. Die so erhaltene Polymermasse wird dann auf eine metallisierte (vorzugsweise aluminisierte) Trägerfolie aus einem geeigneten Kunststoff, insbesondere PETP aufgebracht und z.B. 20 Minuten auf 100°C erhitzt. Als fertiges Produkt erhält man eine transparente, elastische Folie.

Das erfindungsgemäße Hydrogel weist gegenüber Hydrogelen auf Synthesepolymer-Basis und Biopolymer-Basis eine überraschend große Wasserdampfdurchlässigkeit, sowie Elastizität und Flexibilität auf. Ferner nimmt das erfindungsgemäße Hydrogel zügig Wundsekret über einen vergleichsweise langen Zeitraum auf und ist in seinem Absorptionsverhalten dem handelsüblichen Hydrogel auf Basis eines synthetischen Polymeren deutlich überlegen, das schon nach vergleichsweise kurzer Zeit seine maximale Absorption erreicht. Die Kombination von Absorption und hoher Wasserdampfdurchlässigkeit der erfindungsgemäßen Hydrogele erzeugt auf der Wunde ein konstantes Feuchtmilieu, das zu einer beschleunigten Heilung führt. Die erfindungsgemäßen Hydrogele sind darüber hinaus auch transparent und ermöglichen so eine Beobachtung des Wundheilungsverlaufs, so daß bei schwach sezernierenden Wunden der Verbandwechsel in längeren Intervallen erfolgen kann.

Den bekannten Hydrogelen auf Basis von Biopolymeren sind die erfindungsgemäßen Hydrogele - neben Transparenz, Elastizität und Flexibilität vor allem dadurch überlegen, daß sie sich im Wundsekret nicht auflösen.

Die Erfindung wird durch das folgende Beispiel erläutert.

Beispiel 1:

In einem 1 l-Kolben wird ein Gemisch aus 70 g Glycerin, 30 g Gelatine, 30 g Wasser und 5,4 g Copolymer aus Acrylsäure und Natriumacrylat (18 %ige Lösung in Glycerin/Wasser 3:1) unter ständigem Rühren bei 95°C homogenisiert. Der geschmolzenen homogenen Masse werden 18 g Titanacetylacetonat in Form einer 10%igen alkoholischen Lösung zugesetzt.

Eine aluminisierte PETP-Folie wird mit der so gefertigten Polymermasse beschichtet und 20 min auf 100°C erhitzt. Als fertiges Produkt erhält man eine transparente, elastische Folie, deren Eigenschaften in der Tabelle angegeben sind.

Beispiel 2:

Zu der im Beispiel 1 beschriebenen geschmolzenen Masse werden 30 g Aluminiumacetylacetonat in Form einer 5 %igen Ethylacetatlösung zugemischt. Die Eigenschaften des nach der Beschichtung und Trocknung (20 min bei 100°C) erhaltenen Hydrogels zeigt die folgende Tabelle.

Beispiel 3:

Ähnlich wie im Beispiel 1 wird ein Gemisch aus 60 g Glycerin, 10 g 1,2-Propandiol, 35 g Gelatine, 45 g Wasser und 6,2 g Copolymer aus β-Acryloyloxypropionsäure und Kalium-β-Acryloyloxypropionat (15%ige Lösung in Glycerin/Wasser 4:1) unter ständigem Rühren bei 95°C homogenisiert. Die geschmolzene Masse wird mit 4,5 g Melamin-Formaldehyd-Harz abgemischt und wie im Beispiel 1 zum fertigen Produkt bearbeitet. Die Prüfwerte sind ebenso der Tabelle zu entnehmen.

Zu Vergleichszwecken wurden Hydrogelfilme auf Biopolymerbasis, sowie auf Basis eines synthetisches Polymeren herangezogen, deren Eigenschaften ebenfalls in der folgenden Tabelle angeben sind.

| Produkt-Bezeichnung | Dicke (mm) | Farbe | Transparenz | Elastizität | Flexibilität | maximale Dehnung (%) | Klebrigkeit | WDD (g/m²·24h) | Wasser-aufnahme (24h) (Gew.%) |
|---|---|---|---|---|---|---|---|---|---|
| * Cutinova | 0.23 | farblos | transparent | keine da verstärkt | keine | 45 (Netz reißt) | keine | 39 | 500 |
| ** Varihesive | 1.50 | honig-farben | keine | keine | keine | 60 | klebt stark | 2085 | 360 |
| Beispiel 1 | 0.56 | gelblich | transparent | gut | sehr flexibel | 83 | gute Haftung | 3461 | 483 |
| Beispiel 2 | 0.58 | gelblich | transparent | gut | sehr flexibel | 105 | gute Hafutng | 2603 | 450 |
| Beispiel 3 | 0.60 | gelblich | transparent | gut | sehr flexibel | 210 | gute Haftung | 2477 | 468 |

* Cutinova: Zusammensetzung: Polyvinylalkohol

** Varihesive: Zusammensetzung: Gelatine, Carboxymethylcellulose, klebrigmachende Harze

Die geprüften Hydrogele weisen einen unterschiedlichen Absorptionsverlauf auf, wobei das kommerzielle Hydrogel auf Synthesepolymer-Basis ("Cutinova®") schon nach 2 bis 3 Stunden seine maximale Absorption erreicht.

Im Vergleich zu anderen Hydrogelen zeigt das erfindungsgemäße Hydrogel eine ausgezeichnete Wasserdampfdurchlässigkeit (WDD), die in Kombination mit einer zügigen Wundsekretaufnahme dazu führt,

4

daß unabhängig von der Stärke der Sekretion immer ein konstantes, heilungsförderndes Feuchtmilieu auf der Wunde herrscht.

**Patentansprüche**

**Patentansprüche für folgende Vertragsstaaten : AT, BE, CH, DE, DK, FR, GB, IT, LI, LU, NL, SE**

1.  Vernetzte, wundsekretabsorbierende Hydrogele, dadurch gekennzeichnet, daß sie aus folgenden Bestandteilen aufgebaut sind :

    a) 20 - 70 Gew.-% wenigstens eines mehrwertigen Alkohols, ausgewählt aus der Gruppe bestehend aus Gycerin, Ethylenglykol, Diethylenglykol, 1,2-Propandiol, 1,3-Propandiol, 1,2-Butandiol, 1,3-Butandiol, 2,3-Butandiol, 1,4-Butandiol, Glycerinmonoacetat oder ein Gemisch dieser Alkohole,

    b) 10 - 35 Gew.-% wenigstens eines natürlichen Gelierungsmittels (Biopolymer), ausgewählt aus der Gruppe bestehend aus Collagen, Gelatine, Pektine oder deren Gemische oder ein Gemisch Gelatine/Natriumalginat im Verhältnis von 30:1 bis 5:1,

    c) 0,05 - 12 Gew.-% wenigstens eines unvernetzten Copolymeren aus einer oder mehreren Vinylcarbonsäuren und wenigstens einem ihrer Alkali- oder Ammoniumsalze (synthetisches Polymer),

    d) 0,05 - 10 Gew.-% eines dreidimensionale Netzwerke aus bio- und synthetischen Polymeren erzeugenden Vernetzungsmittels, und

    e) 0 - 50 Gew.-% Wasser oder physiologische Kochsalzlösung .

2.  Vernetzte, wundsekretabsorbierende Hydrogele nach Anspruch 1, dadurch gekennzeichnet, daß das Verhältnis Polyvinylcarbonsäure/ Polyvinylcarbonsäuresalz im Copolymeren c) im Bereich von 10:1 bis 1:10 liegt.

3.  Vernetzte, wundsekretabsorbierende Hydrogele nach Anspruch 1, dadurch gekennzeichnet, daß die Vinylcarbonsäure des Copolymeren c) Acrylsäure, Methacrylsäure oder $\beta$-Acryloyloxypropionsäure, Vinylessigsäure, Maleinsäure, Fumarsäure, Crotonsäure, Aconitsäure und/oder Itaconsäure ist.

4.  Vernetzte, wundsekretabsorbierende Hydrogele nach Anspruch 1, dadurch gekennzeichnet, daß das Vernetzungsmittel d) aus der Gruppe der Metallchelate, Orthotitansäureester, Epoxide, Aziridine, Triazine oder Melamin-Formaldehyd-Harze ausgewählt ist.

5.  Vernetzte, wundsekretabsorbierende Hydrogele nach Anspruch 1, dadurch gekennzeichnet, daß das Vernetzungsmittel die Vernetzung von Biopolymer mit synthetischen Polymer zu bevorzugt dreidimensionalen Netzwerken bewirkt.

**Patentansprüche für folgende Vertragsstaaten : ES, GR**

1.  Verfahren zur Herstellung von vernetzten wundsekretabsorbierenden Hydrogelen, dadurch gekennzeichnet, daß

    a) 20 bis 70 Gew.-% wenigstens eines mehrwertigen Alkohols, ausgewählt aus der Gruppe bestehend aus Glycerin, Ethylenglykol, Diethylenglykol, 1,2-Propandiol, 1,3-Propandiol, 1,2-Butandiol, 1,3-Butandiol, 2,3-Butandiol, 1,4-Butandiol, Glycerinmonoacetat oder ein Gemisch dieser Alkohole,

    b) 10 bis 35 Gew.-% wenigstens eines natürlichen Gelierungsmittels (Biopolymer), ausgewählt aus der Gruppe, bestehend aus Collagen, Gelatine, Pektine oder deren Gemische oder ein Gemisch Gelatine/Natriumalginat im Verhältnis von 30:1 bis 5:1,

    c) 0,05 bis 12 Gew.-% wenigstens eines unvernetzten Copolymeren aus einer oder mehreren Vinylcarbonsäuren und wenigstens einem ihrer Alkali- oder Ammoniumsalze (synthetisches Polymer),

    d) 0,05 bis 10 Gew.-% eines dreidimensionale Netzwerke aus bio- und synthetischen Polymeren erzeugenden Vernetzungsmittels, und

    e) 0 bis 50 Gew.-% Wasser oder physiologische Kochsalzlösung

    gegebenenfalls unter Rühren und Erwärmen homogenisiert, die erhaltene Polymermasse zu einer Folie verformt und diese Folie gegebenenfalls durch Erhitzen getrocknet wird.

**2.** Verfahren nach Anspruch 1, <u>dadurch gekennzeichnet,</u> daß das Verhältnis Polyvinylcarbonsäure/Polyvinylcarbonsäuresalz im Copolymeren c) im Bereich von 10:1 bis 1:10 liegt.

**3.** Verfahren nach Anspruch 1, <u>dadurch gekennzeichnet,</u> daß die Vinylcarbonsäure des Copolymeren c) Acrylsäure, Methacrylsäure oder $\beta$-Acryloyloxypropionsäure, Vinylessigsäure, Maleinsäure, Fumarsäure, Crotonsäure, Aconitsäure und/oder Itaconsäure ist.

**4.** Verfahren nach Anspruch 1, <u>dadurch gekennzeichnet,</u> daß das Vernetzungsmittel d) aus der Gruppe der Metallchelate, Orthotitansäureester, Epoxide, Aziridine, Triazine oder Melamin-Formaldehyd-Harze ausgewählt ist.

**5.** Verfahren nach Anspruch 1, <u>dadurch gekennzeichnet,</u> daß das Vernetzungsmittel die Vernetzung von Biopolymer mit synthetischen Polymer zu bevorzugt dreidimensionalen Netzwerken bewirkt.

**Claims**
**Claims for the following Contracting States : AT, BE, CH, DE, DK, FR, GB, IT, LI, LU, NL, SE**

**1.** Cross-linked, wound secretion-absorbing hydrogels, characterized in that they are built up of the following components:
a) 20 to 70%-wt of at least one multivalent alcohol selected from the group consisting of glycerol, ethylene glycol, diethylene glycol, 1,2-propanediol, 1,3-propanediol, 1,2-butanediol, 1,3-butanediol, 2,3-butanediol, 1,4-butanediol, glycerol monoacetate, or a mixture of these alcohols,
b) 10 to 35%-wt of at least one natural gelling agent (biopolymer) selected from the group consisting of collagen, gelatin, pectins, or their mixtures, or a mixture of gelatin/sodium alginate in the ratio of 30:1 to 5:1,
c) 0.05 to 12%-wt of at least one uncross-linked copolymer of one or more vinylcarboxylic acids and at least one of their alkali or ammonium salts (synthetic polymer),
d) 0.05 to 10%-wt of a cross-linking agent creating three-dimensional networks of biopolymers and synthetic polymers,
e) 0 to 50%-wt of water or physiological saline.

**2.** The cross-linked, wound secretion-absorbing hydrogels according to claim 1, characterized in that the ratio polyvinylcarboxylic acid/polyvinylcarboxylic acid salt in copolymer c) is in the range of 10:1 to 1:10.

**3.** The cross-linked, wound secretion-absorbing hydrogels according to claim 1, characterized in that the vinylcarboxylic acid of copolymer c) is acrylic acid, methacrylic acid, or $\beta$-acryloyloxypropionic acid, vinylacetic acid, maleic acid, fumaric acid, crotonic acid, aconitic acid, and/or itaconic acid.

**4.** The cross-linked, wound secretion-absorbing hydrogels according to claim 1, characterized in that the cross-linking agent d) is selected from the group consisting of metal chelates, orthotitanic acid esters, epoxides, aziridines, triazines, or melamine-formaldehyde resins.

**5.** The cross-linked, wound secretion-absorbing hydrogels according to claim 1, characterized in that the cross-linking agent causes the cross-linking of biopolymers with synthetic polymers to form, preferably three-dimensional, networks.

**Claims for the following Contracting States : ES, GR**

**1.** Process for the production of cross-linked, wound secretion-absorbing hydrogels, characterized in that
a) 20 to 70%-wt of at least one multivalent alcohol selected from the group consisting of glycerol, ethylene glycol, diethylene glycol, 1,2-propanediol, 1,3-propanediol, 1,2-butanediol, 1,3-butanediol, 2,3-butanediol, 1,4-butanediol, glycerol monoacetate, or a mixture of these alcohols,
b) 10 to 35%-wt of at least one natural gelling agent (biopolymer) selected from the group consisting of collagen, gelatin, pectins, or their mixtures, or a mixture of gelatin/sodium alginate in the ratio of 30:1 to 5:1,
c) 0.05 to 12%-wt of at least one uncross-linked copolymer of one or more vinylcarboxylic acids and at least one of their alkali or ammonium salts (synthetic polymer),

d) 0.05 to 10%-wt of a cross-linking agent creating three-dimensional networks of biopolymers and synthetic polymers,

e) 0 to 50%-wt of water or physiological saline are homogenised, optionally under stirring and addition of heat, the resulting polymer mass is formed to a film and this film is dried, optionally by heating.

2. The process according to claim 1, characterized in that the ratio polyvinylcarboxylic acid/polyvinylcarboxylic acid salt in copolymer c) is in the range of 10:1 to 1:10.

3. The process according to claim 1, characterized in that the vinylcarboxylic acid of copolymer c) is acrylic acid, methacrylic acid, or $\beta$-acryloyloxypropionic acid, vinylacetic acid, maleic acid, fumaric acid, crotonic acid, aconitic acid, and/or itaconic acid.

4. The process according to claim 1, characterized in that the cross-linking agent d) is selected from the group consisting of metal chelates, orthotitanic acid esters, epoxides, aziridines, triazines, or melamine-formaldehyde resins.

5. The process according to claim 1, characterized in that the cross-linking agent causes the cross-linking of biopolymers with synthetic polymers to form, preferably three-dimensional, networks.

**Revendications**
**Revendications pour les Etats contractants suivants : AT, BE, CH, DE, DK, FR, GB, IT, LI, LU, NL, SE**

1. Hydrogels réticulés, absorbant les sécrétions des blessures ou plaies, caractérisés en ce qu'ils sont constitués des composants qui suivent :

(a) 20 à 70% en poids d'au moins un alcool polyhydroxylé, choisi dans le groupe formé par la glycérine, l'éthylèneglycol, le diéthylèneglycol, le 1,2-propanediol, le 1,3-propanediol, le 1,2-butanediol, le 1,3-butanediol, le 2,3-butanediol, le 1,4-butanediol, le monoacétate de glycérine, ou un mélange de ces alcools;

b) 10 à 35% en poids d'un agent de gélification naturel (biopolymère), choisi dans le groupe formé par le collagène, les gélatines, les pectines, ou leurs mélanges, ou un mélange de gélatine/alginate de sodium dans le rapport de 30:1 à 5:1;

c) 0,05 à 12% en poids d'au moins un copolymère non réticulé d'un ou plusieurs acides vinylcarboxyliques et au moins un de leurs sels de métaux alcalins ou d'ammonium (polymère synthétique);

d) 0,05 à 10% en poids d'un réseau tridimensionnel formé d'un agent de réticulation engendrant des biopolymères et des polymères synthétiques, et

e) 0 à 50% en poids d'eau ou d'une solution de chlorure de sodium physiologique.

2. Hydrogels réticulés, absorbant les sécrétions des plaies ou blessures suivant la revendication 1, caractérisés en ce que le rapport poly(acide vinylcarboxylique)/poly(sel d'acide vinylcarboxylique) dans le copolymère c) varie de 10:1 à 1:10.

3. Hydrogels réticulés, absorbant les sécrétions des plaies ou blessures suivant la revendication 1, caractérisés en ce que l'acide vinylcarboxylique du copolymère c) est l'acide acrylique, l'acide méthacrylique, ou l'acide $\beta$-acryloyloxypropionique, l'acide vinylacétique, l'acide maléique, l'acide fumarique, l'acide crotonique, l'acide aconitique et/ou l'acide itaconique.

4. Hydrogels réticulés, absorbant les sécrétions des plaies ou blessures suivant la revendication 1, caractérisés en ce que l'agent de réticulation d) est choisi dans le groupe formé par les chélates de métaux, les esters de l'acide orthotitanique, les époxydes, les aziridines, les triazines ou les résines de mélamine-formaldéhyde.

5. Hydrogels réticulés, absorbant les sécrétions des plaies ou blessures suivant la revendication 1, caractérisés en ce que l'agent de réticulation assure la réticulation du biopolymère avec le polymère synthétique en réseaux avantageusement tridimensionnels.

**Revendications pour les Etats contractants suivants : ES, GR**

1. Procédé de préparation d'hydrogels réticulés, absorbant les sécrétions des plaies ou blessures, caractérisé en ce que l'on homogénéise :

   (a) 20 à 70% en poids d'au moins un alcool polyhydroxylé, choisi dans le groupe formé par la glycérine, l'éthylèneglycol, le diéthylèneglycol, le 1,2-propanediol, le 1,3-propanediol, le 1,2-butanediol, le 1,3-butanediol, le 2,3-butanediol, le 1,4-butanediol, le monoacétate de glycérine, ou un mélange de ces alcools;

   b) 10 à 35% en poids d'un agent de gélification naturel (biopolymère), choisi dans le groupe formé par le collagène, les gélatines, les pectines, ou leurs mélanges, ou un mélange de gélatine/alginate de sodium dans le rapport de 30:1 à 5:1;

   c) 0,05 à 12% en poids d'au moins un copolymère non réticulé d'un ou plusieurs acides vinylcarboxyliques et au moins un de leurs sels de métaux alcalins ou d'ammonium (polymère synthétique);

   d) 0,05 à 10% en poids d'un réseau tridimensionnel formé d'un agent de réticulation engendrant des biopolymères et des polymères synthétiques, et

   e) 0 à 50% en poids d'eau ou d'une solution de chlorure de sodium physiologique,

   éventuellement sous agitation et chauffage, on transforme la masse polymérique obtenue en une feuille et on sèche cette feuille éventuellement sous chauffage.

2. Procédé suivant la revendication 1, caractérisé en ce que le rapport poly(acide vinylcarboxylique)/poly-(sel d'acide vinylcarboxylique) dans le copolymère c) varie de 10:1 à 1:10.

3. Procédé suivant la revendication 1, caractérisés en ce que l'acide vinylcarboxylique du copolymère c) est l'acide acrylique, l'acide méthacrylique, ou l'acide $\beta$-acryloyloxypropionique, l'acide vinylacétique, l'acide maléique, l'acide fumarique, l'acide crotonique, l'acide aconitique et/ou l'acide itaconique.

4. Procédé suivant la revendication 1, caractérisés en ce que l'agent de réticulation d) est choisi dans le groupe formé par les chélates de métaux, les esters de l'acide orthotitanique, les époxydes, les aziridines, les triazines ou les résines de mélamine-formaldéhyde.

5. Procédé suivant la revendication 1, caractérisés en ce que l'agent de réticulation assure la réticulation du biopolymère avec le polymère synthétique en réseaux avantageusement tridimensionnels.